(19) Europäisches Patentamt
European Patent Office
Office européen des brevets

(11) Publication number: 0 311 009 A2

# (12) EUROPEAN PATENT APPLICATION

(21) Application number: 88116392.7

(22) Date of filing: 04.10.88

(51) Int. Cl.[4]: G03C 1/10 , //C07C157/09, C07C109/087

(30) Priority: 05.10.87 JP 250909/87
31.12.87 JP 336565/87

(43) Date of publication of application: 12.04.89 Bulletin 89/15

(84) Designated Contracting States: DE GB IT NL

(71) Applicant: KONICA CORPORATION
26-2 Nishishinjuku 1 chome
Shinjuku-ku Tokyo(JP)

(72) Inventor: Habu, Takeshi
Konica Corporation 1 Sakura-machi
Hino-shi Tokyo(JP)
Inventor: Uesawa, Yutaka
Konica Corporation 1 Sakura-machi
Hino-shi Tokyo(JP)
Inventor: Usagawa, Yasushi
Konica Corporation 1 Sakura-machi
Hino-shi Tokyo(JP)
Inventor: Ishii, Fumio
Konica Corporation 1 Sakura-machi
Hino-shi Tokyo(JP)
Inventor: Kida, Shuji
Konica Corporation 1 Sakura-machi
Hino-shi Tokyo(JP)

(74) Representative: Türk, Gille, Hrabal
Bruckner Strasse 20
D-4000 Düsseldorf 13(DE)

## (54) Silver halide photographic lightsensitive material.

(57) A silver halide photographic light-sensitive material capable of forming a high contrast image. The photographic material contains at least one compound represented by the following Formulas I or II:

EP 0 311 009 A2

Xerox Copy Centre

Formula I

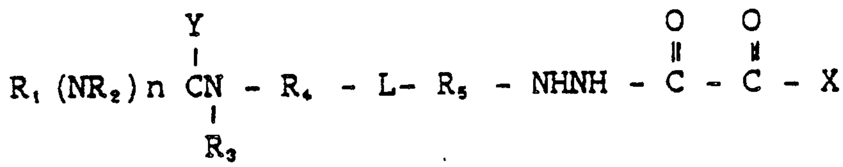

Formula II

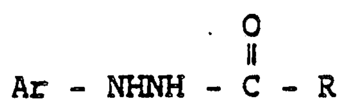

# SILVER HALIDE PHOTOGRAPHIC LIGHT-SENSITIVE MATERIAL

## FIELD OF THE INVENTION

This invention relates to a silver halide photographic light-sensitive material and, particularly, to a silver halide photographic light-sensitive material capable of obtaining high contrast photographic images.

## BACKGROUND OF THE INVENTION

Heretofore, silver halide photographic light-sensitive materials have widely been used in photomechanical processes. One such photomechanical process includes the step of converting a continuous tone original into a halftone dot image. That is, this process includes the step of converting the individual density variations of a continuous tone original into an aggregate of halftone dots having various areas each proportionally corresponding to the continuous tone densities.

Through the above-mentioned conversion step, a silver halide photographic light-sensitive material having high contrast photographic characteristics has been used to photograph an original through a crossover line screen or a contact screen and the light-sensitive material has then been developed, so that a halftone dot image has been formed.

As described in Japanese Patent Publication Open to Public Inspection (hereinafter called Japanese Patent O.P.I. Publication) No. 106244-1981 and U.S. Patent No. 4,686,167, for the purpose of endowing an image with halftone characteristics, silver halide photographic light-sensitive materials have been prepared including a hardener, such as a hydrazine compound, silver halide grains capable of effectively displaying the half-tone characteristics of the above-mentioned compound, and other photographic additives in suitable combination. The silver halide photographic light-sensitive materials thus prepared are stable as light-sensitive materials and are also capable of providing a high contrast photographic image even when processed with a rapid-processable developer.

However, silver halide photographic light-sensitive materials such as those mentioned above have had a problem in that, when converting a continuous tone original into a halftone dot image, a sand-shaped or pin-point shaped fog, that is, the so-called "black-pepper fog", has been incurred in the halftone dots of the image, damaging the quality of the halftone dot image. In attempting to solve the above-mentioned problem, various kinds of stabilizers or inhibitors each having a heteroatom have been added. It is difficult to say that such measures have always been effective.

## SUMMARY OF THE INVENTION

The instant invention has been accomplished to solve the above-described problem.

It is, therefore, an object of the invention to provide a silver halide photographic light-sensitive material capable of displaying halftone photographic characteristics, inhibiting fog produced in a halftone dot image and displaying high contrast photographic characteristics. The invention also provides a method for achieving these objectives.

The above-mentioned object of the invention can be accomplished with a silver halide photographic light-sensitive material comprising at least one compound selected from the group consisting of those compounds represented by Formulas I and II, as follows:

Formula I

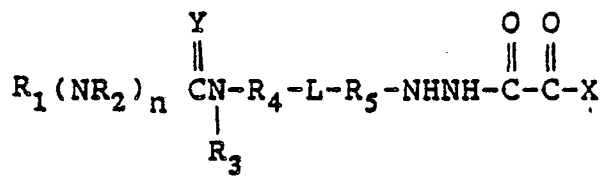

wherein, in Formula I:

$R_1$ and $R_2$ each are a hydrogen atom, or an optionally substituted alkyl, phenyl, naphthyl, cyclohexyl, pyridyl or pyrrolidyl group;

$R_3$ is a hydrogen atom or an optionally substituted benzyl, alkoxy or alkyl group;

$R_4$ and $R_5$ each are a divalent aromatic group;

X is $-NR_6R_7$ or $-OR_8$;

$R_6$, $R_7$ and $R_8$ each are a hydrogen atom or an optionally substituted alkyl, phenyl or naphthyl group;

Y is a sulfur atom or an oxygen atom;

L is a divalent linkage group; and

n is an integer of 0 or 1;

Formula II

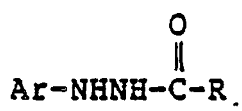

wherein, in Formula II:

Ar represents an aryl group containing at least one ballast group and/or at least one group accelerating adsorption to silver halide; and

R is a substituted alkyl group, the substituent thereof, which itself may optionally be substituted, is at least one group selected from the group consisting of alkoxy, aryloxy, heterocyclic oxy, mercapto, alkylthio, arylthio, heterocyclic thio, alkylsulfonyl, arylsulfonyl, heterocyclic sulfonyl, acyl, cyano, chloro, bromo, fluoro, alkoxycarbonyl, aryloxycarbonyl, carboxy, carbamoyl, alkylcarbamoyl, arylcarbamoyl, amino, alkylamino, arylamino, acylamino, alkoxycarbonylamino, aryloxycarbonylamino, acyloxy, alkylaminocarbonyloxy, arylaminocarbonyloxy, sulfo, sulfamoyl, alkylsulfamoyl and arylsulfamoyl groups; and

wherein, in said Formulas I and II, one or both of the hydrogen atoms in the -NHNH- group is optionally substituted with a substituent.

The invention also provides a method of adding the Formula I and/or Formula II compounds to a silver halide photographic light-sensitive material.

## DETAILED DESCRIPTION OF THE INVENTION

The component materials of the silver halide photographic light-sensitive materials relating to this invention are so constituted as to contain therein at least one compound represented by the aforegiven Formulas I or II. The inclusion of one or both of these compounds allows the light-sensitive materials of the invention to display high contrast photographic characteristics, while inhibiting pin-point fog occurrence, in a halftone dot image.

In the aforegiven Formula I,

$R_1$ and $R_2$ each are a hydrogen atom; or an optionally substituted alkyl group such as a methyl, ethyl,

butyl, dodecyl, 2-hydroxypropyl, 2-cyanoethyl or 2-chloroethyl group; an optionally substituted phenyl, naphthyl, cyclohexyl, pyridyl or pyrrolidyl group, such as a phenyl, p-methylphenyl, naphthyl, $\alpha$ -hydroxynaphthyl, cyclohexyl, p-methylcyclohexyl, pyridyl, 4-propyl-2-pyridyl, pyrrolidyl or 4-methyl-2-pyrrolidyl group;

$R_3$ is a hydrogen atom or an optionally substituted benzyl, alkoxy or alkyl group, such as a benzyl, p-methylbenzyl, methoxy, ethoxy, ethyl or butyl group;

$R_4$ and $R_5$ each are a divalent aromatic group, such as a phenylene or naphthylene group;

Y is a sulfur or oxygen atom;

L is a divalent linkage group, such as $-SO_2CH_2CH_2NH-$, $-SO_2NH-$, $-OCH_2CH_2CONH-$,$-OCH_2SO_2NH-$, -O- or -CH = N-;

X is $-NR_6R_7$ or $-OR_8$;

$R_6$, $R_7$ and $R_8$ each are a hydrogen atom or an optionally substituted alkyl group such as a methyl, ethyl or dodecyl group, an optionally substituted phenyl group, such as a phenyl, p-methylphenyl or p-methoxyphenyl group, or an optionally substituted naphthyl group, such as an $\alpha$-naphthyl or $\beta$ -naphthyl group;

n is an integer of 0 or 1; and

Y is, preferably, a sulfur atom when X is $-OR_8$.

In Formula II, Ar represents an aryl group containing at least one ballast group and/or at least one group accelerating adsorption to silver halide.

The ballast groups preferably include those popularly used in immobile photographic additives such as couplers and so forth. Such ballast groups as mentioned above have not less than 8 carbon atoms and are relatively inert to photographic reactivity. Those ballast groups may be selected from groups such as alkyl, alkoxy, phenyl, alkylphenyl, phenoxy, and alkylphenoxy groups, for example.

The groups accelerating adsorption to silver halide may include, for example, those given in U.S. Patent No. 4,385,108, such as thiourea, thiourethane, heterocyclic thio amido, mercapto heterocyclic, triazole, imidazole, thiazole and other such groups.

R in Formula II represents a substituted alkyl group. Such alkyl groups include, for example, any of those in the straight-chain, branched-chain or cyclic form, such as methyl, ethyl, propyl, butyl, isopropyl, pentyl, cyclohexyl and other such groups.

The substituents introduced into these alkyl groups include, for example, groups such as alkoxy such as methoxy, ethoxy and so forth, aryloxy such as phenoxy, p-chlorophenoxy and so forth, heterocyclic oxy such as pyridyloxy and so forth, mercapto, alkylthio such as methylthio, ethylthio and so forth, arylthio such as phenylthio, p-chlorophenylthio and so forth, heterocyclic thio such as pyridylthio, pyrimidylthio, thiadiazolylthio and so forth, alkylsulfonyl such as methanesulfonyl, butanesulfonyl and so forth, arylsulfonyl such as benzenesulfonyl and so forth, heterocyclic sulfonyl such as pyridylsulfonyl, morpholinosulfonyl and so forth, acyl such as acetyl, benzoyl and so forth, cyano, chloro, bromo, fluoro, alkoxycarbonyl such as ethoxycarbonyl, methoxycarbonyl and so forth, aryloxycarbonyl such as phenoxycarbonyl and so forth, carboxy, carbamoyl, alkylcarbamoyl such as N-methyl-carbamoyl, N,N-dimethylcarbamoyl and so forth, arylcarbamoyl such as N-phenylcarbamoyl and so forth, amino, alkylamino such as methylamino, N,N-dimethylamino and so forth, arylamino such as phenylamino, naphthylamino and so forth, acylamino such as acetylamino, benzoylamino and so forth, alkoxycarbonylamino such as ethoxycarbonylamino and so forth, aryloxycarbonylamino such as phenoxycarbonylamino and so forth, acyloxy such as acetyloxy, benzoyloxy and so forth, alkylaminocarbonyloxy such as methylaminocarbonyloxy and so forth, arylaminocarbonyloxy such as phenylaminocarbonyloxy and so forth, sulfo, sulfamoyl, alkylsulfamoyl such as methylsulfamoyl and so forth, arylsulfamoyl such as phenylsulfamoyl and so forth, and the like groups.

In Formulas I and/or II, one or both of the H atoms of the -NHNH- group, that is, the hydrogen atoms of the hydrazine group, may optionally be substituted with substituents including, for example, sulfonyl groups, such as methanesulfonyl, toluenesulfonyl and other such groups, acyl groups such as acetyl, trifluoroacetyl and other such groups, oxalyl groups such as ethoxalyl and other such groups.

Exemplary compounds represented by the aforegiven Formulas I and II are given as follows. The compounds represented by Formulas I and II which may be used in the invention, however, are not, of course, limited to these exemplary compounds.

1 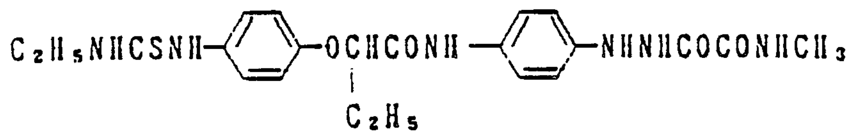

2 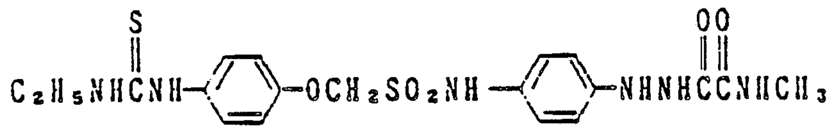

3 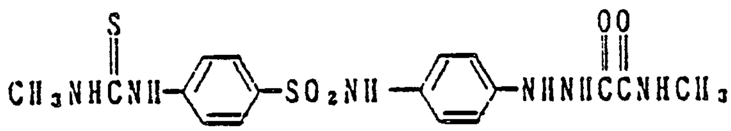

4 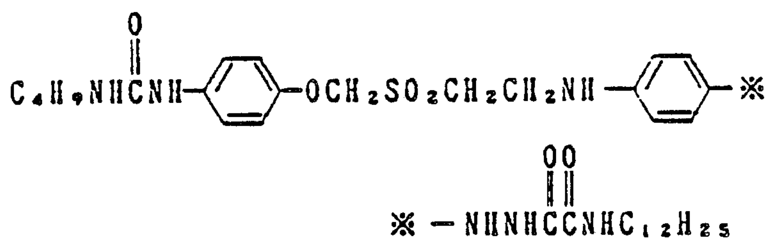

5 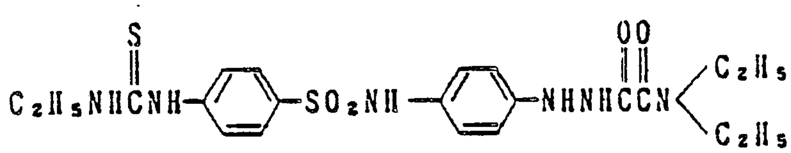

6 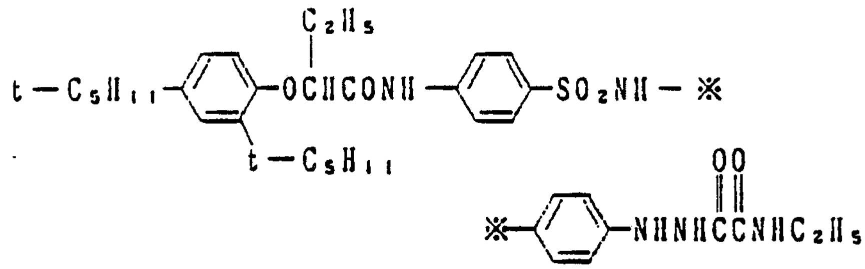

7 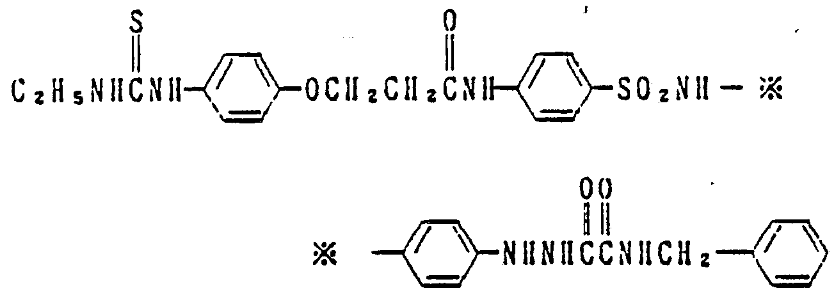

8 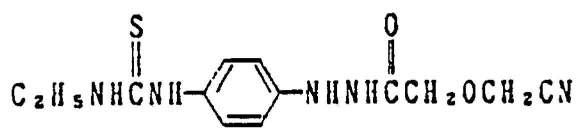

9 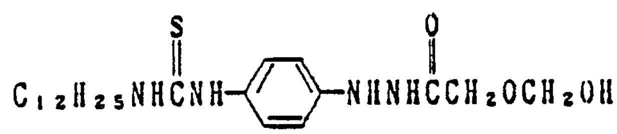

10 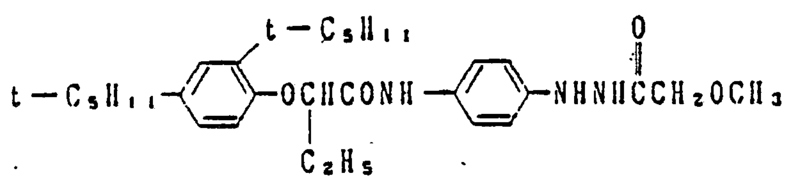

11 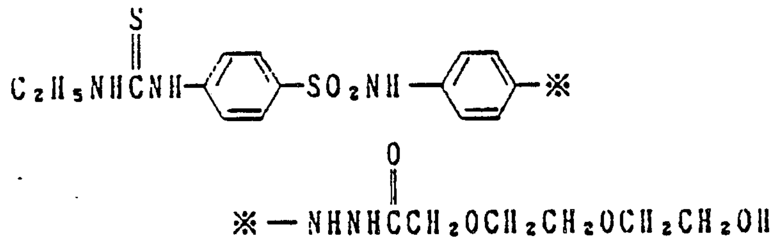

12 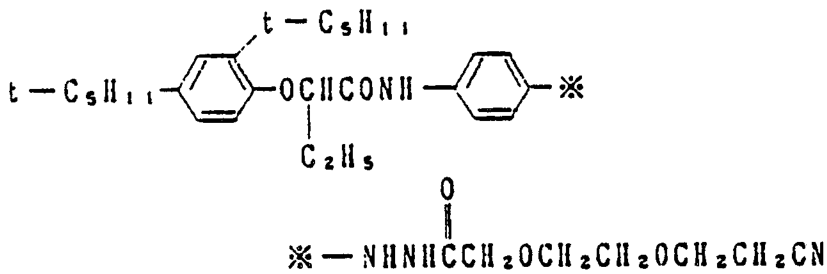

13 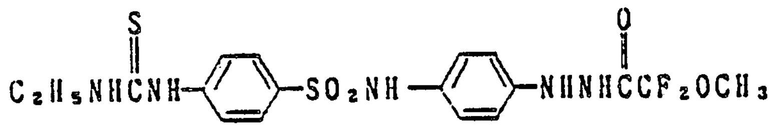

14 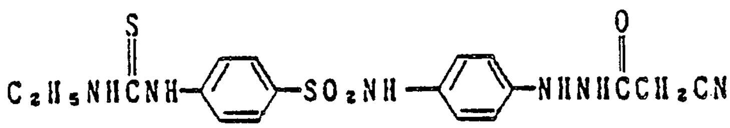

15 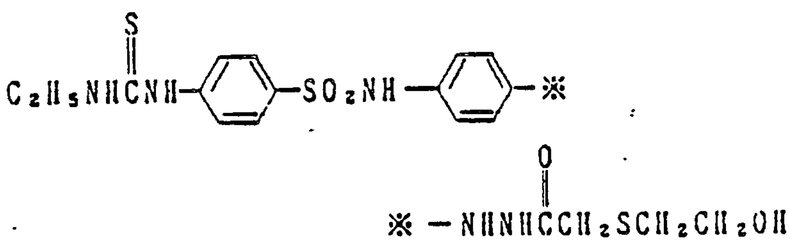

16 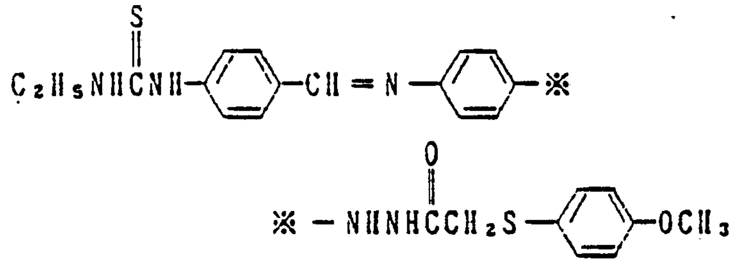

17 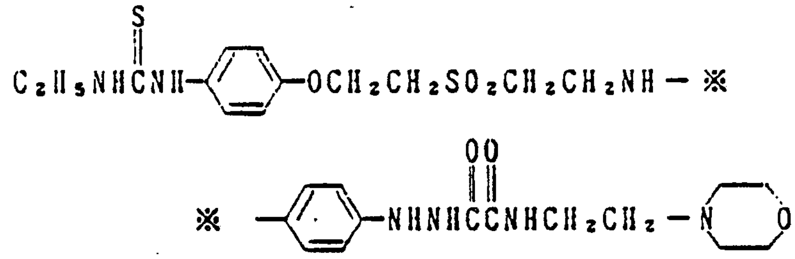

18 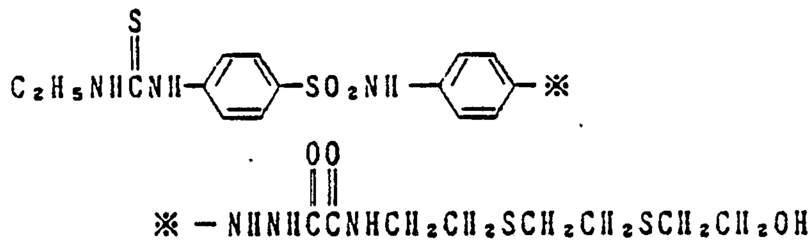

19 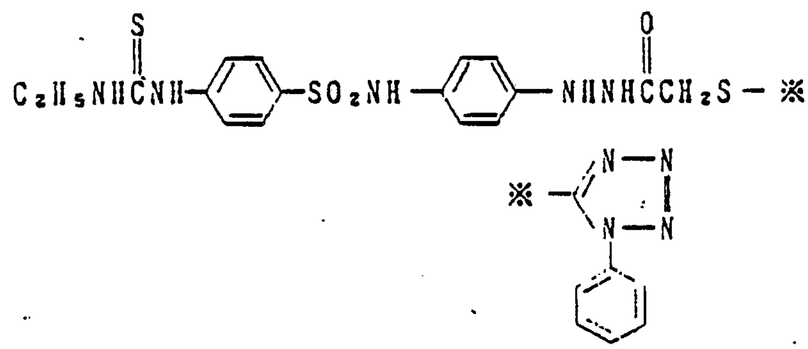

20 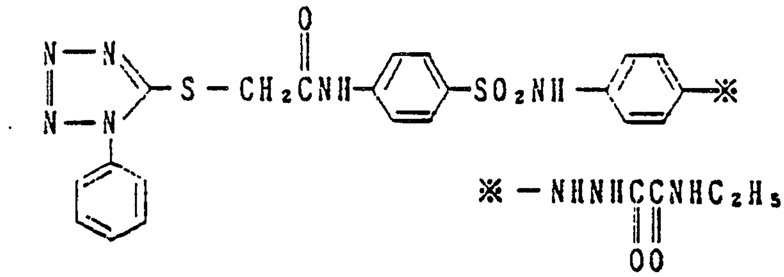

21 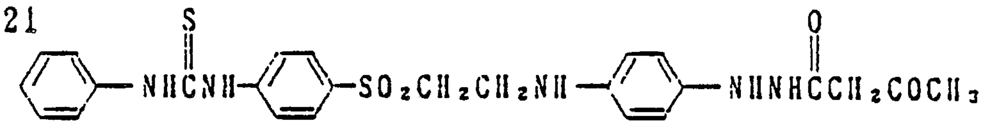

22 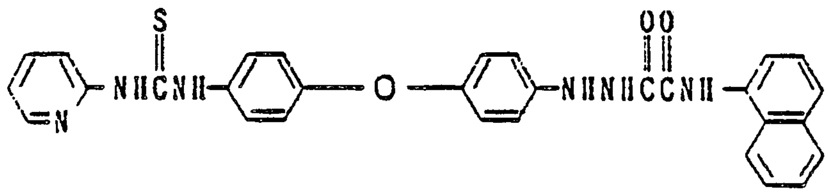

23 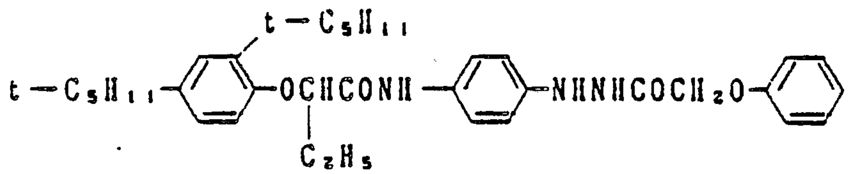

24 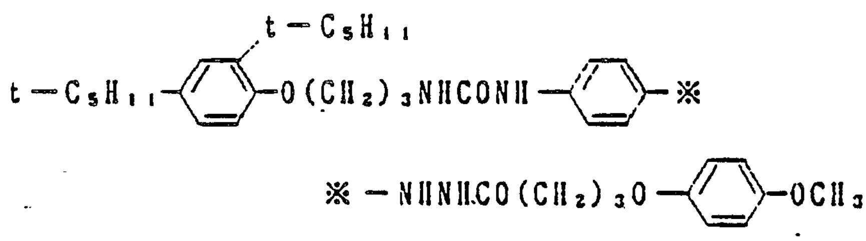

25 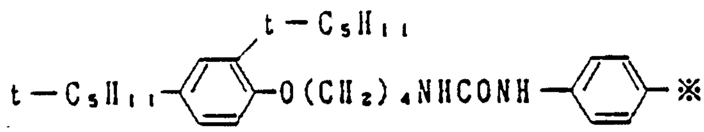

※ − $NHNHCOCH_2OCH_3$

26 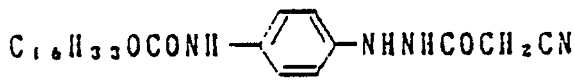

27 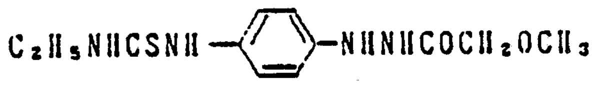

28 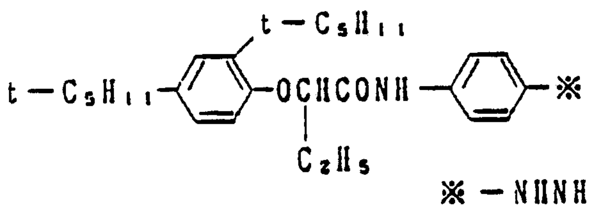

※ − $NHNHCOCH_2CH_2COOC_2H_5$

29 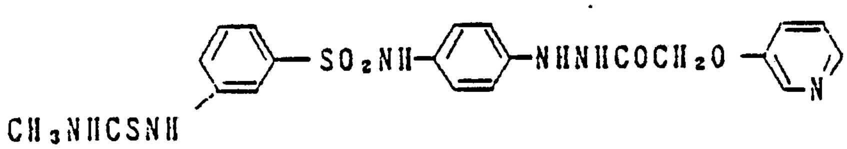

30 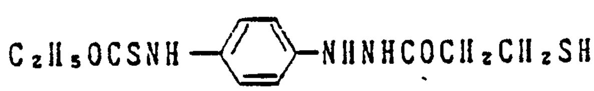

31 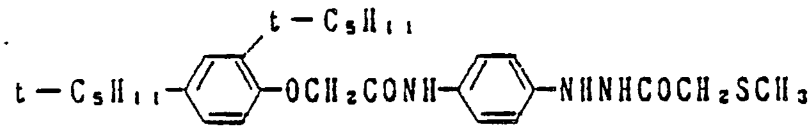

32 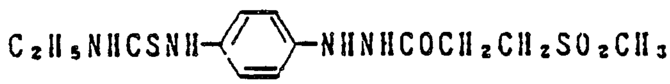

33 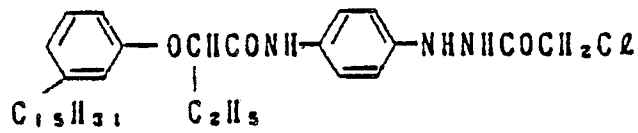

34 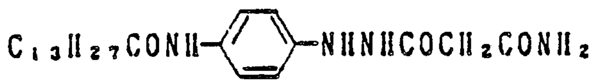

35 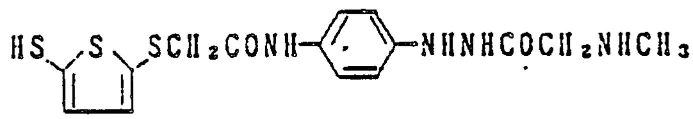

36 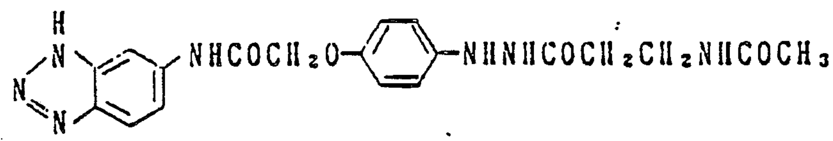

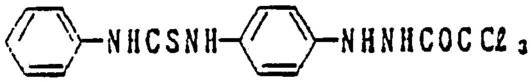

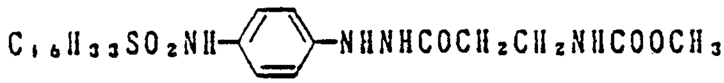

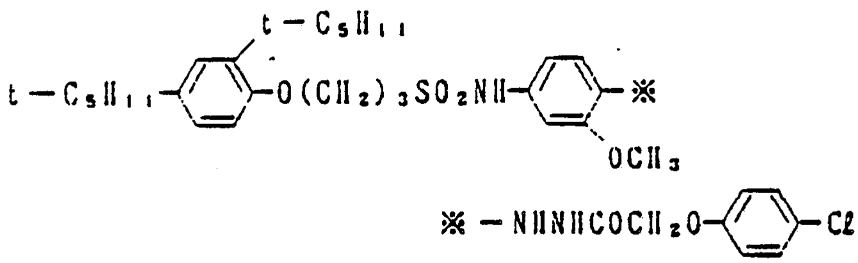

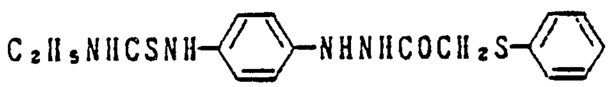

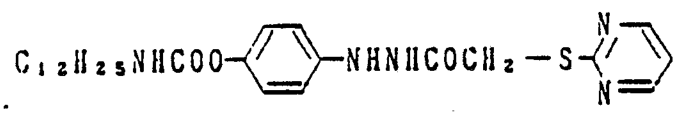

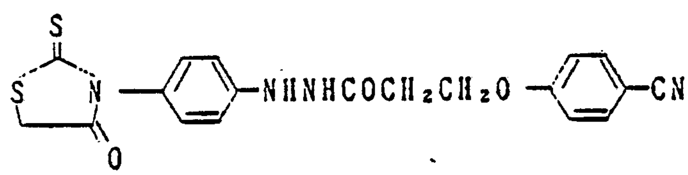

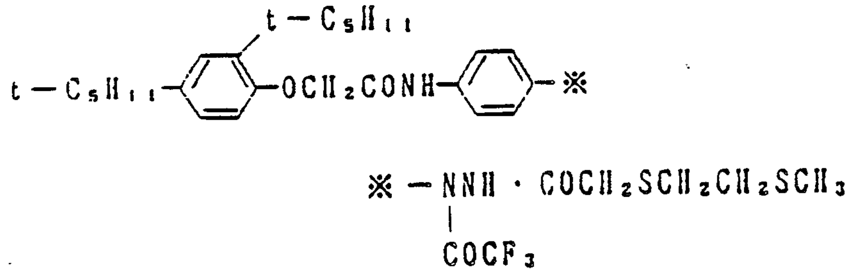

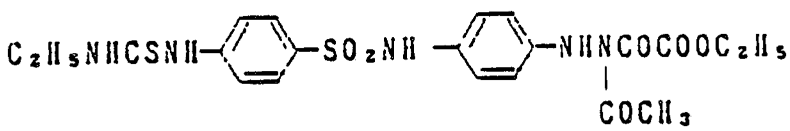

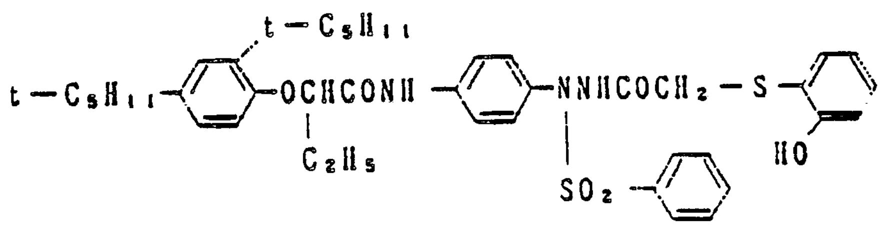

The compounds of the invention may be prepared by methods known to those skilled in the art.

Examples illustrating the synthesis of Compounds 1, 3 and 27 of the above-given exemplary compounds are described below.

## Synthesis Examples

### Synthesis of Compound 1:

The synthesis scheme was as follows.

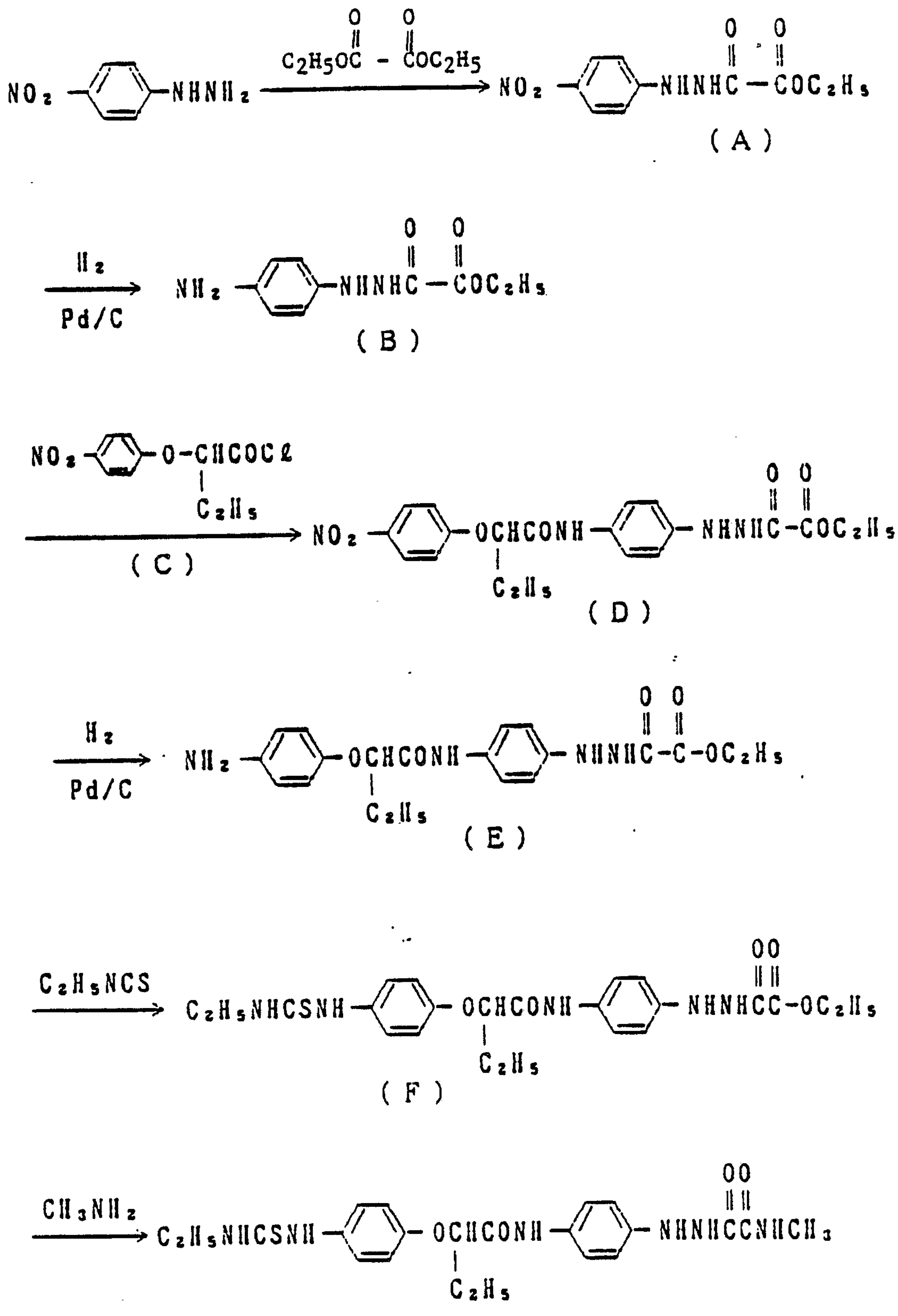


**Compound 1**

That is, 153 g of 4-nitrophenyl hydrazine and 500 ml of diethyl oxalate were mixed together and were then refluxed for one hour. While the reaction proceeded ethanol was removed, and the reacted matter was finally cooled so as to deposit crystals. The resulting crystals were filtrated and washed with petroleum ether several times so as to be recrystallized. 50 g of the resulting crystals of Compound (A) were dissolved

with heating in 1000 ml of methanol, and were then reduced in an $H_2$ atmosphere, applied with a pressure of 50 psi in the presence of Pd/C (palladium/carbon), so that Compound (B) was obtained.

22 g of Compound (B) were dissolved in a solution of 200 ml of acetonitrile and 16 g of pyridine. Into the resulting solution, an acetonitrile solution containing 24 g of Compound (C) was dropped at room temperature. After the insoluble matters were filtrated away, the resulting filtrate was condensed, recrystallized and refined, so that 31 g of Compound (D) were obtained. 30 g of the resulting Compound (D) were hydrogenated in the same manner as above, so that 20 g of Compound (E) were obtained.

10 g of Compound (E) were dissolved in 100 ml of acetonitrile and 3.0 g of ethylisothiocyanate were added. The resulting solution was refluxed for one hour. After the solvents were distilled off, the resulting matter was recrystallized and refined, so that 7.0 g of Compound (F) were obtained. 5.0 g of Compound (F) were dissolved in 50 ml of methanol and 8 ml of an aqueous 40% methylamine solution were added and stirred. After the methanol was somewhat condensed, the deposited solid matters were taken out, recrystallized and refined, so that Compound 1 was obtained.

<u>Synthesis of Compound 3:</u>

The synthesis scheme was as follows.

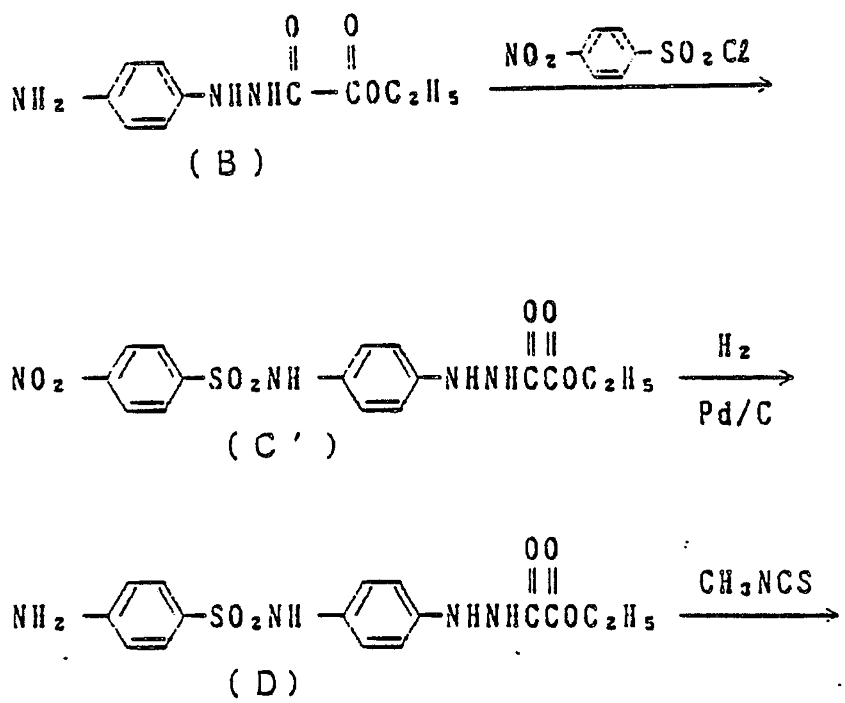


(B)

(C′)

(D)

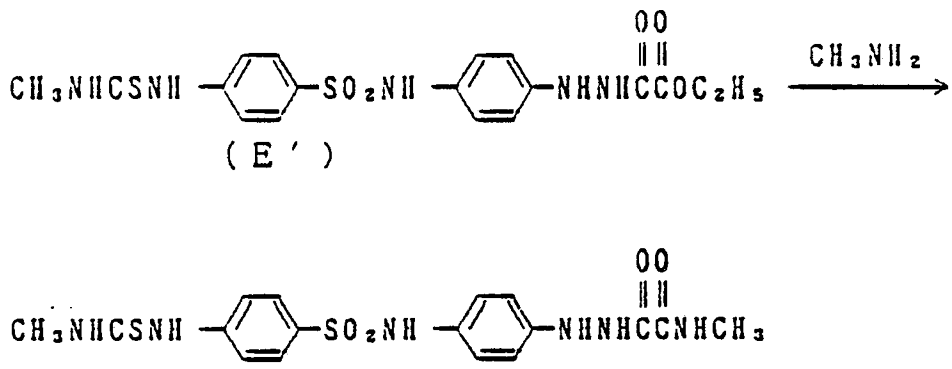


(E′)

Compound 3

That is, 22 g of Compound (B) were dissolved in 200 ml of pyridine and 22 g of p-nitrobenzenesulfonyl chloride were added with stirring. After the reacted mixture was poured into water, the deposited solid matters were taken out, so that Compound (C′) was obtained. According to the synthesis scheme, the Compound (C′) was treated in the same reaction as in Compound 1, so that Compound 3 was obtained.

Synthesis of Compound 27:

The synthesis scheme was as follows.

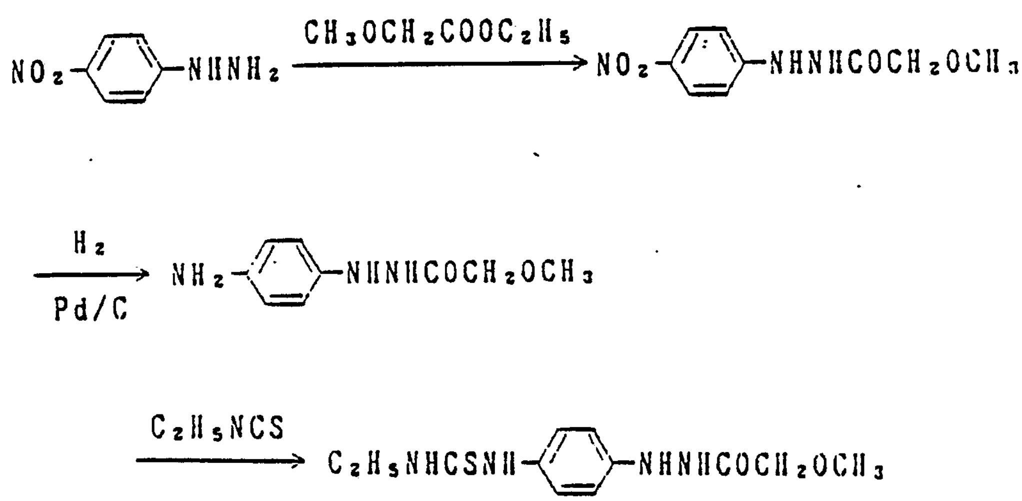


Compound 27

Compound 27 was obtained according to the synthesis method of Compound 1.

The silver halide photographic light-sensitive materials of the invention are to contain at least one compound represented by the aforegiven Formulas I or II. The amount of these compounds contained therein is preferably from $5x10^{-7}$ to $5x10^{-1}$ mol per mol of silver halide used and, more preferably, within the range of from $5x10^{-5}$ to $1x10^{-2}$ mol per mol of silver halide used.

The silver halide photographic light-sensitive materials of the invention may have at least one silver halide emulsion layer. At least one of such silver halide emulsion layers may be provided on one side of a

support in one embodiment, and to both sides thereof in another embodiment of the invention. Also, the silver halide emulsions may be coated on the support either directly or by the interposition of other layers such as a hydrophilic colloidal layer not containing any silver halide emulsion. Further, above the silver halide emulsion layer, a hydrophilic colloidal layer serving as a protective layer may be coated. Two or more separate silver halide emulsion layers of different speeds may be coated, such as a high speed layer and a low speed layer. In this case, an interlayer comprising a hydrophilic colloid may be arranged between these silver halide emulsion layers. An interlayer between the silver halide emulsion layer and the protective layer may also be provided. That is, if desired, non-light-sensitive hydrophilic colloidal layers such as an interlayer, a protective layer, an antihalation layer, a backing layer and so forth may be provided.

In the silver halide photographic light-sensitive materials of the invention containing the compounds represented by Formulas I or II, it is preferable to include one or both of these compounds in the hydrophilic colloidal layers of the light-sensitive materials, and more preferable to include them in the silver halide emulsion layers and/or the hydrophilic colloidal layers adjacent to the silver halide emulsion layers.

The compounds represented by Formulas I or II need not be included in the silver halide emulsion layers, and may, in fact, be included in any of the other layers. For example, these compounds may be included in any of the other hydrophilic colloidal layers multilayered over the emulsion layers.

The most preferable embodiment of the invention is that the compounds represented by Formulas I or II are included in the silver halide emulsion layers and hydrophilic colloids comprised of gelatin or a gelatin derivative.

Next, the methods of placing the compounds represented by Formulas I or II into hydrophilic colloidal layers will be described. These methods include, for example, a method where the compounds are dissolved in an appropriate amount of water and/or an organic solvent and the resulting solution is added into a hydrophilic colloidal layer. In another method, the compounds are dissolved in an organic solvent and the resulting solution is dispersed in a hydrophilic colloidal matrix such as gelatin, a gelatin derivative or the like and the resulting dispersion is then added into a hydrophilic colloidal layer. In a further method, the compounds are dispersed into a latex and the resulting dispersion is added into a hydrophilic colloidal layer, and so forth. When embodying the invention, any of these methods may be used. Even when adding these compounds independently, preferable image characteristics may be obtained. These compounds may be contained in combination in a desired proportion.

In addition to the above-mentioned methods, another method which may be employed is one where the compounds represented by Formulas I or II are dissolved in appropriate organic solvents including, for example, water, alcohols such as methanol or ethanol, ethers, esters and so forth, and the resulting solution is coated directly by an overcoating method or the like to the portion which will be the outermost layer of the silver halide emulsion layers of the silver halide photographic light-sensitive material, so that the compounds may be contained in the silver halide photographic light-sensitive material.

As described above, in a preferable embodiment of the invention, the compounds represented by Formulas I or II are added into silver halide emulsion layers and, in another preferable embodiment of the invention, the compounds are added directly or through an interlayer into the hydrophilic colloidal layer adjacent to a hydrophilic colloidal layer containing a silver halide emulsion.

The compounds represented by Formulas I or II need not be present in the silver halide photographic light-sensitive material as initially prepared. At least one of these compounds may be added during subsequent treatment, such as development, of these materials. For example, at least one compound of Formula I or II may be added to the developing solution employed. Thus, as long as a silver halide photographic light-sensitive material is at some point prior to the completion of the development process contacted with at least one Formula I or II compound, the beneficial effects of the invention may be obtained.

Next, the silver halides, which may be used in the silver halide photographic light-sensitive materials of the invention, will be described. With respect to these silver halides, those having any compositions may be used. For example, they may include silver chloride, silver chlorobromide, silver chloroiodobromide, silver bromide such as pure silver bromide or silver iodobromide. The average grain size of these silver halides should preferably be within the range of from 0.05 to 0.5 micron and should more preferably be within the range of from 0.10 to 0.40 micron.

The grain size distribution of the silver halides used in the invention may be freely selected. The grain size distribution is preferably adjusted to yield a monodisperse size distribution. The degree of monodispersion, defined below, may preferably be within the range of 1 to 30 and more preferably within the range of 5 to 20.

Herein, the degree of monodispersion will be defined by the following formula.

$$\text{Degree of monodispersion} = \sqrt{\frac{\sum (\bar{r}-ri)^2 ni}{\sum ni}} \div \bar{r} \times 100$$

The values of the degree of monodispersion are defined as the 100-fold value of a value obtained by dividing a standard deviation value of grain sizes by an average grain size value. The grain sizes of the silver halides may conveniently be expressed in terms of the edge length of a cubic crystal grain.

When embodying the invention, silver halide grains having at least a two-layered or more multilayered type structure may be employed, for example, silver chlorobromide grains having silver bromide cores and silver chloride shells. In this case, any layers may contain an iodide and the iodide contents should preferably not be more than 5 mol%.

Further, when preparing a silver halide emulsion, the sensitivity and gradation thereof may be controlled by adding a rhodium salt. The point of time when adding the rhodium salt should preferably be at the point of time when the emulsion grains are formed. However, it may also be added either when a chemical ripening is carried out or when an emulsion coating solution is prepared. Such a rhodium salt may not only be a simple salt but may also be a complex salt. The typical rhodium salts used therein include, for example, rhodium chloride, rhodium trichloride, rhodium ammonium chloride and so forth.

The amount of rhodium salts to be added may be varied according to the sensitivity and gradation desired. An amount within the range of from $10^{-9}$ mol to $10^{-4}$ mol per mol of silver used is particularly effective.

Other inorganic compounds such as iridium salts, platinum salts, thallium salts, cobalt salts, gold salts and so forth may be used alone, in combination with each other or in combination with a rhodium salt. Among these compounds, the iridium salts may often be used, particularly for the purpose of providing high illuminance characteristics. The iridium salts should preferably be used in an amount within the range of from $10^{-9}$ mol to $10^{-4}$ mol per mol of silver used.

Further, the silver halides may be sensitized with a variety of chemical sensitizers. Such sensitizers include, for example, active gelatin, sulfur sensitizers such as sodium thiosulfate, allyl thiocarbamide, thiourea, allyl isothiacyanate and so forth, selenium sensitizers such as N,N-dimethylselenourea, selenourea and so forth, reduction sensitizers such as triethylene tetramine, stannous chloride and so forth, a variety of noble metal sensitizers typically including potassium chloroaurite, potassium aurithiocyanate, potassium chloroaurate, 2-aurosulfobenzothiazole methyl chloride, ammonium chloropalladate, potassium chloroplatinate, sodium chloropalladite and so forth. These sensitizers may be used independently or in combination. When using a gold sensitizer, ammonium thiocyanate may further be used to serve as an assistant.

In the invention, an emulsion containing surface latent image type silver halide grains may be suitably used. The term 'a surface latent image type grain', as used herein, means a grain which is so prepared as to make the sensitivity obtained by processing with what is known in the art as a 'surface developer' higher than the sensitivity obtained by processing with what is known in the art as an 'internal developer'.

The silver halide emulsions which may be used in the invention may be stabilized or made to inhibit the occurrence of fog by making use of mercapto compounds such as 1-phenyl-5-mercaptotetrazole or 2-mercaptobenzothiazole, benzotriazole compounds such as 5-bromobenzotriazole or 5-methylbenzotriazole, benzimidazole compounds such as 6-nitrobenzimidazole, and so forth.

The silver halide emulsions which may be used in the invention may also contain a sensitizing dye, a plasticizer, an antistatic agent, a surface active agent, a hardening agent and so forth.

In a hydrophilic colloidal layer of the invention to which compounds represented by Formulas I or II may be added, gelatin may suitably be used as the hydrophilic colloidal layer, although any other hydrophilic colloid may also be used.

The supports which may be used in an embodiment of the invention include, for example, a baryta paper, a polyethylene-coated paper, polypropylene synthetic paper, glass plate, cellulose acetate film, cellulose nitrate film, polyester film such as those films of polyethylene terephthalate, and so forth. These supports may suitably be selected to satisfy the purposes for which the silver halide photographic light-sensitive material is intended.

When developing the silver halide photographic light-sensitive materials of the invention, the following developing agents, for example, may be used.

Typical examples of unsaturated, hydroxyl containing developing agents such as the HO-(CH=CH)n-OH type developing agents, include hydroquinone and, also, catechol, pyrogallol and so forth.

Unsaturated, hydroxyl and amine containing developing agents such as the HO-(CH=CH)n-$NH_2$ type developing agents include, for example, an ortho- and para-aminophenol or aminopyrazolone and, also, N-methyl-p-aminophenol, N-β-hydroxyphenyl aminoacetate, 2-aminonaphthol and so forth.

Heterocyclic ring type developing agents include, for example, a 3-pyrazolidone, such as 1-phenyl-3-pyrazolidone, 1-phenyl-4,4-dimethyl-3-pyrazolidone, 1-phenyl-4-methyl-4-hydroxymethyl-3-pyrazolidone, 1-phenyl-4-methyl-4-hydroxymethyl-3-pyrazolidone and so forth.

In addition to the above, other developing agents may effectively be employed including developing agents such as those given in, for example, T.H. James, The Theory of the Photographic Process, 4th Ed., pp. 291-334; and Journal of the American Chemical Society, Vol. 73, p.3,100, 1951; and so forth. These developing agents may be used either independently or in combination. It may be preferable to use them in combination. When using the developing agent independently, hydroquinone is preferably used. When using them in combination, hydroquinone and 1-phenyl-3-pyrazolidone or N-methyl-p-aminophenol are preferably used in combination.

The benefits of the invention are not lessened even if the developer for developing the light-sensitive materials of the invention contains a preservative including, for example, a sulfite such as sodium sulfite, potassium sulfite or the like. Hydroxylamine or hydrazide compounds may also be used as the preservatives. Additionally, a pH adjusting function and a buffering function may be provided to the above-mentioned developer by making use of caustic alkali, carbonate alkali, amine or the like such as those used in general black-and-white developers and, it is further allowed to add freely therein an inorganic development inhibitor such as potassium bromide or the like, an organic development inhibitor such as benzotriazole, a metal-ion scavenger such as ethylenediaminetetraacetic acid or the like, a development accelerator such as methanol, ethanol, benzyl alcohol, polyalkylene oxide or the like, a surface active agent such as sodium alkylarylsulfonate, natural saponin, sugars, the alkyl esters of the above-given compounds, or the like, a hardening agent such as glutaraldehyde, formalin, glyoxal or the like, an ion-strength adjusting agent such as sodium sulfate or the like.

The developers which may be used in the invention may further contain an alkanol amine or a glycol so as to serve as an organic solvent.

## EXAMPLES

Some examples of the invention will now be described. The invention shall not, of course, be limited to only the following examples.

### Example 1

Samples were prepared by adding the exemplified compounds represented by Formulas I or II and the comparative compounds (as given in the following Table 1), in the following manner, into the silver halide emulsion layers of photographic light-sensitive materials, respectively.

### Preparation of silver halide photographic light-sensitive materials

Samples No. 1 through No. 23 were prepared in the following manner. A polyethylene terephthalate film support was used having a thickness of 100 microns, both sides of which were provided with a subbing layer of 0.1 micron in thickness. The silver halide emulsion layer having the following Formula 1 was coated over the subbing layer provided onto one side of the film support so that the amount of gelatin was to be 1.5 $g/m^2$ and the amount of silver was to be 3.3. $g/m^2$ and the protective layer having the following Formula 2 was coated thereover so that the amount of gelatin was to be 1.0 $g/m^2$. Over the subbing layer provided on the other side of the film support, a backing layer was coated according to the following Formula 3 so that the amount of gelatin was to be 3.5 $g/m^2$ and the protective layer having the following Formula 4 was coated thereover so that the amount of gelatin was to be 1 $g/m^2$, respectively.

Formula 1 (Composition of the silver halide emulsion layer)

| | |
|---|---|
| Gelatin | 1.5 $g/m^2$ |
| Silver chlorobromide: (AgCl at 60 mol%, AgBr at 40 mol%, degree of monodispersion = 12) | 3.3 $g/m^2$ |
| Antifogging agent: 4-hydroxy-6-methyl-1,3,3a,7-tetrazaindene | 0.30 $g/m^2$ |
| Compound of the invention, or comparative compound: | Refer to Table 1 |
| Surface active agent: Saponin | 0.1 $g/m^2$ |
| Latex polymer: Polyethyl acrylate | 1 $g/m^2$ |
| Sensitizing dye: Four kinds thereof having the following structural formulas a through d were used in combination | |

Formula a: Regular sensitizing dye

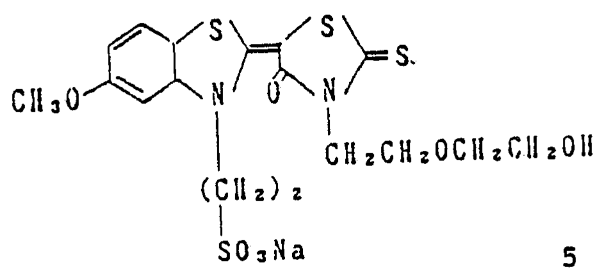

5 $mg/m^2$

Formula b: Ortho sensitizing dyes

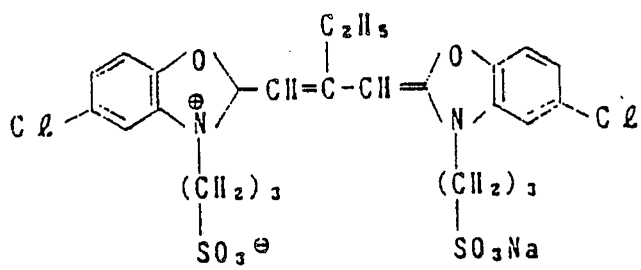

15mg／m²

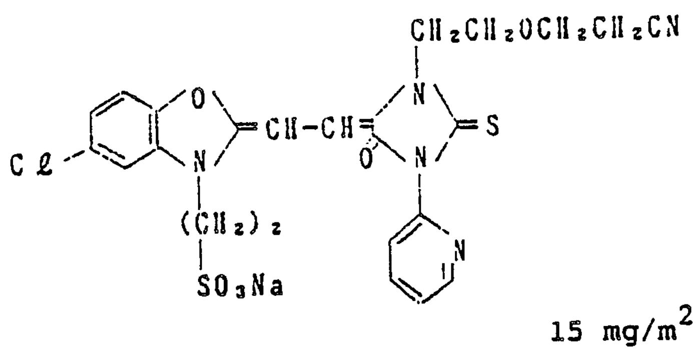

15 mg/m$^2$

Formula c: Panchromatic sensitizing dye

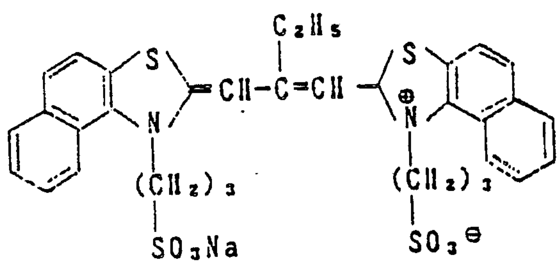

10 mg/m$^2$

Formula d: Infrared sensitizing dye

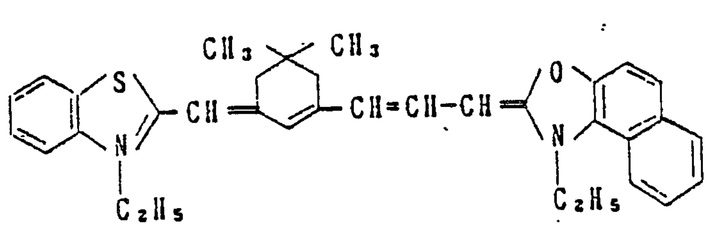

3 mg/m$^2$

| | |
|---|---|
| Development adjusting agent: | |
| Nonylphenoxy polyethylene glycol | 10 mg/$m^2$ |
| 5-methylbenzotriazole | 7 mg/$m^2$ |
| Adenine | 3 mg/$m^2$ |
| Guanine | 2 mg/$m^2$ |
| Urasil | 2 mg/$m^2$ |
| 1-phenyl-5-mercaptotetrazole | 3 mg/$m^2$ |
| Hydroquinone | 100 mg/$m^2$ |
| Phenidone | 10 mg/$m^2$ |

Formula 2 (Composition of Emulsion Protective Layer)

| | |
|---|---|
| Gelatin | 1.0 g/$m^2$ |
| Matting agent: Polymethyl methacrylate having an average particle size of from 3.0 to 5.0 microns | 0.05 g/$m^2$ |
| Surface active agent: Sodium n-dodecylbenzene sulfonate | 0.01 g/$m^2$ |
| Static modifiers: | |
| $C_8F_{17}COONH_4$ | 10 mg/$m^2$ |
| NaCl | 100 mg/$m^2$ |
| LiCl | 30 mg/$m^2$ |
| Stabilizer: 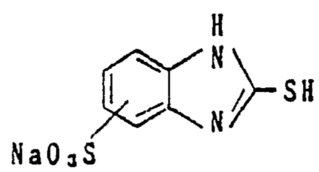 | 5 mg/$m^2$ |
| 1-phenyl-5-mercaptotetrazole | 3 mg/$m^2$ |
| Hardening agent: Formalin | 0.03 g/$m^2$ |

Formula 3 (Composition of Backing Layer)

| | |
|---|---|
| Gelatin | 3.5 g/m$^2$ |
| Dyestuffs: | |
| 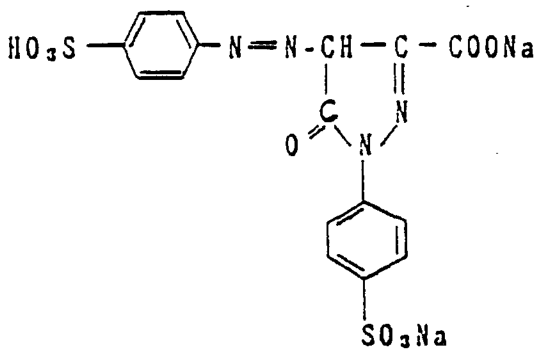 | 1 g/m$^2$ |
| 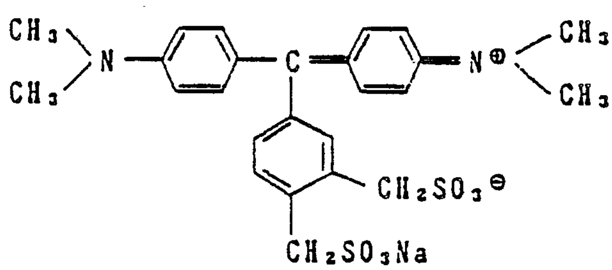 | 1 g/m$^2$ |
| Surface active agent: Saponin | 0.1 g/m$^2$ |
| Hardening agent: Glyoxal | 0.1 g/m$^2$ |

Formula 4 (Composition of Backing Protective Layer)

| | |
|---|---|
| Gelatin | 1 g/m$^2$ |
| Matting agent: | |
| Polymethyl methacrylate having an average particle size of from 3.0 to 5.0 microns | 0.5 g/m$^2$ |
| Surface active agent: | |

| | |
|---|---|
| Sodium p-dodecylbenzene sulfonate | 0.01 $mg/m^2$ |
| $C_8F_{17}SO_2NH(CH_2)_3\overset{(CH_3)_2}{\underset{\oplus}{N}}-CH_2COO^{\ominus}$ | |
| Development adjusters: | |
| 5-nitroindazole | 0.012 $g/m^2$ |
| 5-methylbenzotriazole | 0.02 $g/m^2$ |
| 1-phenyl-5-mercaptotetrazole | 0.005 $g/m^2$ |
| Hardening agent: Formalin | 0.03 $g/m^2$ |

With respect to the samples prepared, the dot quality tests were performed in the following manner.

## Dot Quality Testing Method

Each of the samples was brought into contact with a step wedge partly attached to a contact halftone screen of 150 lines per inch having a dot area of 50% and was then exposed to light emitted from a xenon light source for 5 seconds. The exposed samples were developed under the following conditions with a rapid processing type automatic processor using therein the following developer and fixer. The resulting dot quality of each sample was observed by making use of a magnifier of 100X magnifications. The resulting dot qualities were evaluated with respect to, for example, dot shape and discreteness of the edges thereof, and were ranked from the highest as '5' and so forth as '4', '3', and '2' down to the lowest as '1', respectively. Among those qualities, those ranked as '1' and '2' were on the practically undesirable level.

The fogginess caused in the halftone dots was also evaluated. No black dot effect was found at all in the halftone dots ranked highest as '5' and so forth ranked down as '4', '3', '2', and '1' according to the degree of the black dot effects found in the halftone dots. Among these black dot effects, those ranked as '1' and '2' had substantially large sized black dots and were on the practically undesirable level.

Formula of Developer

(Composition A)

| | |
|---|---|
| Pure water (Ion-exchanged water) | 150 ml |
| Disodium ethylenediaminetetraacetate | 2 g |
| Diethylene glycol | 50 g |
| Potassium sulfite, in an aqueous 55% w/v solution | 100 ml |
| Potassium carbonate | 50 g |
| Hydroquinone | 15 g |
| 5-methylbenzotriazole | 200 mg |
| 1-phenyl-5-mercaptotetrazole | 30 mg |
| Potassium hydroxide | An amount to make the pH of the developer to be a pH of 10.4. |
| Potassium bromide | 3 g |

(Composition B)

| | |
|---|---|
| Pure water (Ion-exchanged water) | 3 ml |
| Diethylene glycol | 50 g |
| Diethylamino-1,2-propanediol | 15 g |
| Disodium ethylenediaminetetraacetate | 25 mg |

| | |
|---|---|
| Acetic acid, in an aqueous 90% solution | 0.3 ml |
| 5-nitroindazole | 110 mg |
| Sodium 2-mercaptobenzimidazole-5-sulfonate | 30 mg |
| 1-phenyl-3-pyrazolidone | 500 mg |

Before using the developer, the above-mentioned Compositions A and B were dissolved in order in 500 ml of water to make 1 liter.

Formula of Fixer

(Composition A)

| | |
|---|---|
| Ammonium thiosulfate, in an aqueous 72.5% w/v solution | 240 ml |
| Sodium sulfite | 17 g |
| Sodium acetate, trihydrate | 6.5 g |
| Boric Acid | 6 g |
| Sodium citrate, dihydrate | 2 g |
| Acetic acid, in an aqueous 90% w/w solution | 13.6 ml |

(Composition B)

| | |
|---|---|
| Pure water (Ion-exchanged water) | 17 ml |
| Sulfuric acid, in an aqueous 50% w/w solution | 4.7 g |
| Aluminium sulfate, an an aqueous 8.1% w/w solution in terms of an $Al_2O_3$ content | 26.5 g |

Before using the fixer, the above-mentioned Compositions A and B were dissolved in 500 ml of water to make 1 liter. The pH value of the fixer was about pH 4.3.

| Development Conditions | | |
|---|---|---|
| Processing Step | Temperature | Time |
| Developing | 38° C | 30 sec. |
| Fixing | 28° C | 20 sec. |
| Washing | At ordinary temperature | 20 sec. |

To the silver halide emulsion layer prepared in the above Formula 1, the following comparative compounds (a) through (c) were added.

Comparative Compound (a)

( a )

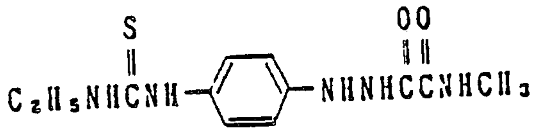

Comparative Compound (b)

( b )

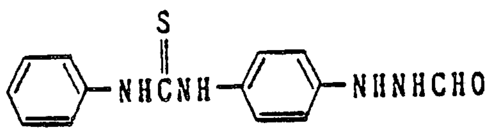

Comparative Compound (c)

( c )

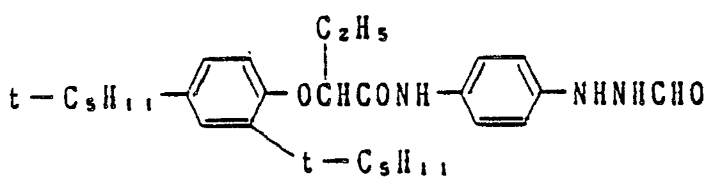

Test Results

With respect to the samples of the invention No. 1 through No. 20 and the samples No. 21 through No. 23 which were prepared by making use of the above-given comparative compounds, Table 1 shows the compounds and the amounts thereof added to the silver halide emulsion layers of the samples. In the table, the compounds represented by Formulas I or II are indicated by the reference numbers of the aforegiven exemplified compounds.

Table 2 shows the results of the dot quality tests of the above-mentioned ranked samples.

As is obvious from Table 2, as far as the dot quality is concerned, the samples No. 1 through No. 20 of the invention resulted in ranks of not lower that '4', and most of them were ranked as '5', while the comparative samples No. 21 through No. 23 resulted in the rank of '3'. Since the ranks of 1 and '2' are not on the level for practical use, it is hard to say that any of the samples No. 21 through No. 23 are excellent in dot quality, while the samples No. 1 through No. 20 of the invention proved to be extremely high and excellent in dot quality.

With respect to the black dot effects which may be regarded as the index of fogginess, the samples No. 1 through No. 20 of the invention were all evaluated and ranked as '5' or '4', that is, they were excellent and resulted in no fogginess. On the other hand, the comparative samples No. 21 through No. 23 were ranked as not higher than '2', proving that they were hardly fit for practical use.

Table 1

| Sample No. | Compound No. | Amount added /Ag 1 mol | Remarks |
|---|---|---|---|
| 1 | 1 | $5x10^{-4}$ mol | Invention |
| 2 | 3 | $5x10^{-4}$ mol | Invention |
| 3 | 10 | $5x10^{-4}$ mol | Invention |
| 4 | 11 | $5x10^{-4}$ mol | Invention |
| 5 | 12 | $5x10^{-4}$ mol | Invention |
| 6 | 13 | $5x10^{-4}$ mol | Invention |
| 7 | 14 | $5x10^{-4}$ mol | Invention |
| 8 | 15 | $5x10^{-4}$ mol | Invention |
| 9 | 16 | $5x10^{-4}$ mol | Invention |
| 10 | 19 | $5x10^{-4}$ mol | Invention |
| 11 | 23 | $5x10^{-4}$ mol | Invention |
| 12 | 25 | $5x10^{-4}$ mol | Invention |
| 13 | 27 | $5x10^{-4}$ mol | Invention |
| 14 | 31 | $5x10^{-4}$ mol | Invention |
| 15 | 32 | $5x10^{-4}$ mol | Invention |
| 16 | 35 | $5x10^{-4}$ mol | Invention |
| 17 | 37 | $5x10^{-4}$ mol | Invention |
| 18 | 39 | $5x10^{-4}$ mol | Invention |
| 19 | 40 | $5x10^{-4}$ mol | Invention |
| 20 | 41 | $5x10^{-4}$ mol | Invention |
| 21 | a | $5x10^{-4}$ mol | Comparative |
| 22 | b | $5x10^{-4}$ mol | Comparative |
| 23 | c | $5x10^{-4}$ mol | Comparative |

Table 2

| Sample No. | Dot quality | Black dot effect | Remarks |
|---|---|---|---|
| 1 | 4 | 4 | Invention |
| 2 | 4 | 4 | Invention |
| 3 | 5 | 5 | Invention |
| 4 | 5 | 5 | Invention |
| 5 | 4 | 5 | Invention |
| 6 | 5 | 5 | Invention |
| 7 | 5 | 5 | Invention |
| 8 | 4 | 5 | Invention |
| 9 | 5 | 5 | Invention |
| 10 | 4 | 5 | Invention |
| 11 | 4 | 5 | Invention |
| 12 | 5 | 5 | Invention |
| 13 | 5 | 4 | Invention |
| 14 | 4 | 5 | Invention |
| 15 | 5 | 5 | Invention |
| 16 | 5 | 5 | Invention |
| 17 | 5 | 5 | Invention |
| 18 | 4 | 4 | Invention |
| 19 | 5 | 5 | Invention |
| 20 | 5 | 5 | Invention |
| 21 | 3 | 2 | Comparative |
| 22 | 3 | 2 | Comparative |
| 23 | 3 | 1 | Comparative |

## Example 3

Samples No. 24 through 33 were prepared in the same manner as in Sample No. 7 and Sample No. 13 of Example 1, except that the degrees of monodispersion (i.e., the uniformity of grain sizes) of the silver halide grains were changed to degrees of from 4 to 40, and the resulting samples were tested.

When the grains were prepared, rhodium in an amount of 8 x $10^{-7}$ mol per mol of Ag and iridium in an amount of 3 x $10^{-7}$ mol per mol of Ag were contained therein by an ordinary method. The silver halide grains were composed of silver chlorobromide grains having a silver chloride content of 98 mol%, and the desensitizing dye having the following chemical structure was added in place of the sensitizing dyes (a), (b), (c), and (d).

Desensitizing dye (in which the total value of the positive and negative potentials obtained by a Polarograph was positive).

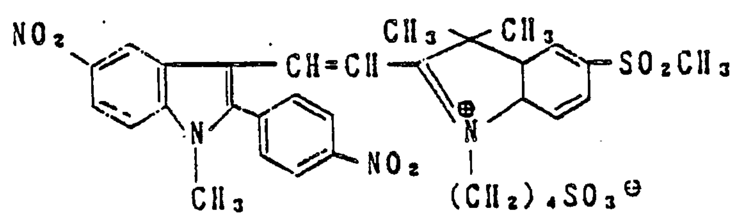

Further, the following filter dyestuff in an amount of 50 mg/$m^2$ was added into the protective layer.

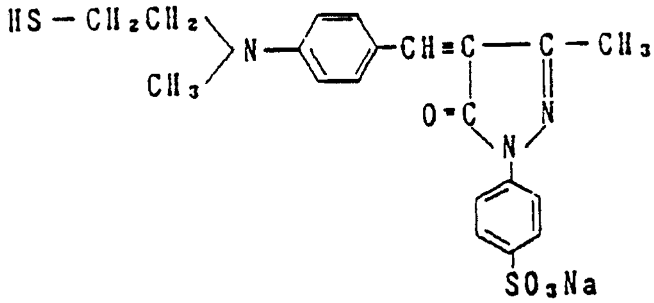

Maximum absorption wavelength
($H_2O$) Max.: 492 nm

Still further, the following UV absorbing dyestuff in an amount of 100 mg/m$^2$ was added thereto.

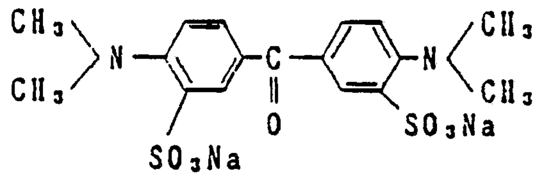

Except where indicated above, the samples of this example were treated in the same manner as in the aforementioned samples No. 7 and No. 13. For example, exemplified compounds No. 14 and No. 27 which are representative of Formulas I or II were used. The degree of monodispersion may be adjusted by suitably adjusting the pAg potential and the supplies of $Ag^{(+)}$ ions and halide ions when preparing grains, in a controlled double jet precipitation method that is an ordinary method.

The photographic characteristics were evaluated after the exposure and developing processes were performed, almost in the same manner as in Example 1, provided that, in this example, the prepared samples were exposed to light by making use of an extra high-pressure mercury vapor lamp with an energy of 5 mJ.

Table 3 shows the results of the evaluations. Samples No. 24 through No. 33 displayed the dot qualities of from 4.5 to 5 and the black dot effect of from 4.5 to 5. It was found that the dot qualities were high, and the fogginess were also very few.

Table 3

| Sample No. | Compound No. | Degree of monodispersion of silver halide grains | Photographic characterisitcs | |
|---|---|---|---|---|
| | | | Dot quality | Black dot |
| 24 | 14 | 40 | 4.5 | 4.5 |
| 25 | 14 | 35 | 4.7 | 4.7 |
| 26 | 14 | 20 | 4.8 | 4.8 |
| 27 | 14 | 10 | 5 | 5 |
| 28 | 14 | 4 | 5 | 5 |
| 29 | 27 | 40 | 4.5 | 4.5 |
| 30 | 27 | 35 | 4.6 | 4.6 |
| 31 | 27 | 20 | 4.8 | 4.8 |
| 32 | 27 | 10 | 5 | 5 |
| 33 | 27 | 4 | 5 | 5 |

## Claims

1. A silver halide photographic light-sensitive material comprising at least one compound selected from the group consisting of those compounds represented by Formulas I and II, as follows:

Formula I

$$R_1(NR_2)_n\underset{R_3}{\overset{Y}{C}}N - R_4 - L - R_5 - NHNH - \overset{O}{\overset{\|}{C}} - \overset{O}{\overset{\|}{C}} - X$$

wherein, in Formula I:
$R_1$ and $R_2$ each represent a hydrogen atom, or a substituted or unsubstituted alkyl, phenyl, naphthyl, cyclohexyl, pyridyl, or pyrrolidyl group;
$R_3$ represents a hydrogen atom or a substituted or unsubstituted benzyl, alkoxy or alkyl group;
$R_4$ and $R_5$ each represent a divalent aromatic group;
X represents $-NR_6R_7$ or $-OR_8$;
$R_6$, $R_7$ and $R_8$ each represent a hydrogen atom or a substituted or unsubstituted alkyl, phenyl or naphthyl group;
Y represents a sulfur atom or an oxygen atom;
L represents a divalent linkage group; and
n represents an integer of 0 or 1;

Formula II

$$Ar - NHNH - \overset{O}{\overset{\|}{C}} - R$$

wherein, in Formula II:
Ar represents an aryl group containing at least one ballast group and/or at least one group accelerating adsorption to silver halide; and
R represents a substituted alkyl group, wherein the substituent thereof may itself optionally be further substituted, and wherein said substituent is at least one group selected from the group consisting of alkoxy, aryloxy, heterocyclic oxy, mercapto, alkylthio, arylthio, heterocyclic thio, alkylsulfonyl, arylsulfonyl, heterocyclic sulfonyl, acyl, cyano, chloro, bromo, fluoro, alkoxycarbonyl, aryloxycarbonyl, carboxy, carbamoyl, alkylcarbamoyl, arylcarbamoyl, amino, alkylamino, arylamino, acylamino, alkoxycarbonylamino, aryloxycarbonylamino, acyloxy, alkylaminocarbonyloxy, arylaminocarbonyloxy, sulfo, sulfamoyl, alkylsulfamoyl, and arylsulfamoyl groups; and
wherein, in said Formulas I and II, one or both of the hydrogen atoms in the -NHNH- group is optionally substituted.

2. The material of claim 1, wherein said ballast group is a group having not less than eight carbon atoms selected from alkyl, alkoxy, phenyl, alkylphenyl, phenoxy and alkylphenoxy groups.

3. The material of claim 1 or 2, wherein said group accelerating adsorption to silver halide is a thiourea, thiourethane, heterocyclic thio amido, mercapto heterocylic, imidazole, thiazole or triazole group.

4. The material of claim 1, 2 or 3, wherein said silver halide photographic light-sensitive material comprises a support having thereon at least one silver halide emulsion layer.

5. The material of claim 4, wherein said at least one Formula I or Formula II compound is contained in said at least one silver halide emulsion layer.

6. The material of claim 5, wherein said at least one Formula I or Formula II compound is contained in said silver halide emulsion layer in an amount within the range of from 5 x $10^{-7}$ mol to 5 x $10^{-1}$ mol per mol of silver halide contained in said silver halide emulsion layer.

7. The material of claim 6, wherein the amount of said compound contained in said silver halide emulsion layer is within the range of from 5 x $10^{-5}$ mol to 5 x $10^{-2}$ mol per mol of silver halide contained in said silver halide emulsion layer.

8. The material of claim 4, wherein said at least one Formula I or Formula II compound is contained in a hydrophilic colloidal layer adjacent to said silver halide emulsion layer.

9. The material of claim 4, wherein silver halide grains contained in said silver halide emulsion layer contain silver iodide in an amount of not more than 5 mol%.

10. The material of claim 4, wherein silver halide grains contained in said silver halide emulsion layer contain a rhodium salt in an amount within the range of from $10^{-9}$ to $10^{-4}$ mol per mol of silver halide.

11. The material of claim 4, wherein silver halide grains contained in said silver halide emulsion layer contain an iridium salt in an amount within the range of from $10^{-9}$ mol to $10^{-4}$ mol per mol of silver halide.

12. The material of claim 1 or claims 1 to 11, wherein said material comprises at least one compound selected from those compounds represented by Formula II.

13. The material of claim 1 or claims 1 to 12, wherein said material comprises silver halide grains which are monodisperse.

14. The material of claim 1 or claims 1 to 13, wherein said material comprises surface latent image type silver halide grains.

15. A method of treating silver halide photographic material comprising the step of contacting said material with at least one compound selected from the group consisting of those compounds represented by Formulas I and II, as follows:

**Formula I**

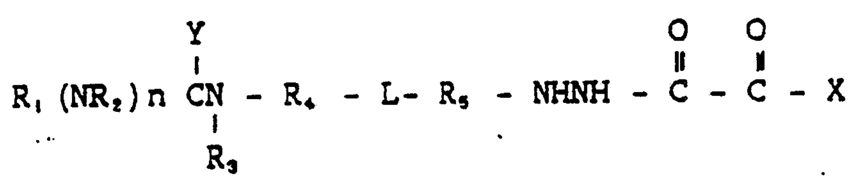

wherein, in Formula I:

$R_1$ and $R_2$ each represent a hydrogen atom, or a substituted or unsubstituted alkyl, phenyl, naphthyl, cyclohexyl, pyridyl, or pyrrolidyl group;

$R_3$ represents a hydrogen atom or a substituted or unsubstituted benzyl, alkoxy or alkyl group;

$R_4$ and $R_5$ each represent a divalent aromatic group;

X represents $-NR_6R_7$ or $-OR_8$;

$R_6$, $R_7$ and $R_8$ each represent a hydrogen atom or a substituted or unsubstituted alkyl, phenyl or naphthyl group;

Y represents a sulfur atom or an oxygen atom;

L represents a divalent linkage group; and

n represents an integer of 0 or 1;

Formula II

$$Ar - NHNH - \overset{\overset{O}{\|}}{C} - R$$

wherein, in Formula II;

Ar represents an aryl group containing at least one ballast group and/or at least one group accelerating adsorpticn to silver halide; and

R represents a substituted alkyl group, wherein the substituent thereof may itself optionally be further substituted, and wherein said substituent is at least one group selected from the group consisting of alkoxy, aryloxy, heterocyclic oxy, mercapto, alkylthio, arylthio, heterocyclic thio, alkylsulfonyl, arylsulfonyl, heterocyclic sulfonyl, acyl, cyano, chloro, bromo, fluoro, alkoxycarbonyl, aryloxycarbonyl, carboxy, carbamoyl, alkylcarbamoyl, arylcarbamoyl, amino, alkylamino, arylamino, acylamino, alkoxycarbonylamino, aryloxycarbonylamino, acyloxy, alkylaminocarbonyloxy, arylaminocarbonyloxy, sulfo, sulfamoyl, alkylsulfamoyl, and arylsulfamoyl groups; and

wherein, in said Formulas I and II, one or both of the hydrogen atoms in the -NHNH- group is optionally substituted.

16. The method of claim 15, wherein said silver halide photographic material is contacted with at least one compound selected from those compounds represented by Formula II.

17. The method of claim 15 or 16, wherein said contacting occurs during the development of said silver halide photographic light-sensitive material and wherein said at least one Formula I or Formula II compound is contained in the developing solution.

18. The method of claim 15, 16 or 17, wherein during the contacting step the compound of Formula I or II is present in an amount effective to inhibit pin-point fog occurrence in a halftone dot image of the developed photographic material.

19. The method of claim 15 or 16 to 18, wherein during the contacting step the compound of Formula I or II is present in an amount in the range of from $5 \times 10^{-7}$ mol to $5 \times 10^{-1}$ mol per mol of silver halide contained in a silver halide emulsion layer.

20. The method of claim 15 or 16 to 19, wherein during the contacting step the compound of Formula I or II is present in an amount in the range of from $5 \times 10^{-5}$ mol to $5 \times 10^{-2}$ mol per mole of silver halide contained in a silver halide emulsion layer.

21. The method of claim 18, wherein the contacting occurs during the development of the photographic material.

22. The method of claim 21, wherein the compound of Formula I or II is contained in the developing solution.

23. The method of claim 15, wherein the contacting occurs during the development of the photographic material.